(19) Europäisches Patentamt
European Patent Office
Office européen des brevets

(11) Publication number: 0 113 029
B1

(12) **EUROPEAN PATENT SPECIFICATION**

(45) Date of publication of patent specification: **19.04.89**

(51) Int. Cl.[4]: **C 07 K 5/12,** C 07 K 7/26, A 61 K 37/02

(21) Application number: **83111747.8**

(22) Date of filing: **24.11.83**

(54) **Dicyclic hexapeptides, processes for their preparation and pharmaceutical compositions containing them.**

(30) Priority: **06.12.82 US 446938**

(43) Date of publication of application: **11.07.84 Bulletin 84/28**

(45) Publication of the grant of the patent: **19.04.89 Bulletin 89/16**

(84) Designated Contracting States: **BE CH DE FR GB IT LI NL SE**

(56) References cited:
**EP-A-0 029 310**
**US-A-4 190 648**

(73) Proprietor: **MERCK & CO. INC.**
**126, East Lincoln Avenue P.O. Box 2000**
**Rahway New Jersey 07065-0900 (US)**

(72) Inventor: **Brady, Stephen F.**
**20 Waterman Avenue**
**Philadelphia, PA. 19118 (US)**

(74) Representative: **Blum, Rudolf Emil Ernst et al**
**c/o E. Blum & Co Patentanwälte Vorderberg 11**
**CH-8044 Zürich (CH)**

EP 0 113 029 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

Somatostatin is a tetradecapeptide incorporating a cyclic dodecapeptide, having the structure:

Ala–Gly–Cys–Lys–Asn–Phe–Phe–Trp–Lys–Thr–Phe–Thr–Ser–Cys–OH

1 2 3 4 5 6 7 8 9 10 11 12 13 14

and has the properties of inhibiting the release of growth hormone, inhibiting the release of insulin and glucagon and reducing gastric secretions. Somatostatin itself has a short duration of action because it is inactivated, *inter alia*, by aminopeptidases and carboxypeptidases present *in vivo*. The problem of the short duration of action has been partially solved in the prior art by preparing derivatives of somatostatin which have low solubility, thus attaining a slow release on subcutaneous injection. Once dissolved, however, the derivatives are no more stable to inactivation by aminopeptidases and carboxypeptidases than somatostatin itself.

Summary of the Invention

The present invention provides for bridged, preferably disulfide bridged cyclic hexapeptides which are derivatives of somatostatin in which, *inter alia*, eight of the ring amino acids are replaced by Cys-Cys or cystathionyl groupings and both of the exocyclic amino acids are removed. The cyclic hexapeptides have to have a further bridge comprising either three methylene groups and one sulfur atom or two methylene groups and two sulfur atoms. Furthermore to the α-carbon atom of one of the amino acids forming the cyclic peptide structure, there is bonded an aliphatic chain comprising 3 to 5 $-CH_2$-groups or 3 $-CH_2$-groups and one sulfur atom in any desired position of the aliphatic chain. Said aliphatic side chain, furthermore, has to have in the ω-position an amino substituent and according to a preferred embodiment the aliphatic side chain comprises 4 $-CH_2$-groups, and accordingly the corresponding amino acid in the cyclic hexapeptide structure which contributes said amino substituted aliphatic side chain is lysine.

Description of the Prior Art

Somatostatin analogs which are cyclic peptides are already described in publications.

In the European patent publication 0 029 310 there are described cyclic hexapeptides which are somatostatin analogs and in which there is bonded to the alpha carbon atom of one of the alpha amino acids of the cyclic structure an aliphatic chain comprising totally 3—5 $-CH_2$-groups or 3 $-CH_2$-Groups and a sulfur atom in the chain, which may be in any position along that chain. Said aliphatic side chain, furthermore, has to have an amino substituent in the ω-position. Preferably said diamino acid which contributes said side chain is lysine.

The cyclic hexapeptides described in said European patent publication, however, differ from the claimed cyclic hexapeptides in that they are free of a further bridge comprising one or two sulfur atoms. For example in said publication no cyclic hexapeptide is disclosed, which comprises two cysteine moieties which would be able to form a corresponding cystine-bridge comprising two $-CH_2$-groups and two sulfur atoms. Therefore, the cyclic hexapeptides disclosed in said publication are not structurally closely related to the inventive compounds.

The cyclic somatostatin analogs described in the U.S. patent 4190648 are as well as cyclic peptides in which totally six alpha amino acids are present in said cyclic structure. One of said amino acids has to be lysine and furthermore two of said amino acids can be cysteine. In this case both cysteine form a further ring closing bridge comprising two methylene groups and two sulfur atoms.

The cyclic hexapeptides of said U.S. patent, however, differ from the inventive cyclic hexapeptides in that the cyclic structure is ring closed by a seventh amino acid, which however is not an alpha amino acid but the 7-amino-heptanoic acid. Therefore, the cyclic peptides disclosed in said U.S. patent are not structurally closely related to the inventive cyclic hexapeptides.

It was the aim of the present invention to provide new somatostatin analogs which have a longer duration of activity than somatostatin and which, furthermore, have a more selective biological activity than somatostatin. Somatostatin inhibits the release of glucagon, the release of growth hormone and the release of insulin and it, furthermore, also inhibits the release of gastric secretions. Accordingly, if somatostatin is used for inhibiting one of said pancreatic functions, respectively the inhibition of the release of gastric acid secretion, then undesired side effects are encountered because of the other inhibitory activities of somatostatin.

The aim of the present invention, accordingly, was to provide a new class of somatostatin analogs having an activity spectrum which differs from the corresponding activity of somatostatin.

It was surprisingly found out that new cyclic hexapeptides which have in their molecule a further sulfur containing aliphatic bridge fulfil the set requirements.

Description of the Invention

The subject of the present invention are now somatostatin analogs, which are cyclic hexapeptides which comprise a further aliphatic group which contains either one sulfur atom and three methylene groups or two sulphur atoms and two methylene groups.

Said new compounds have the following formula I

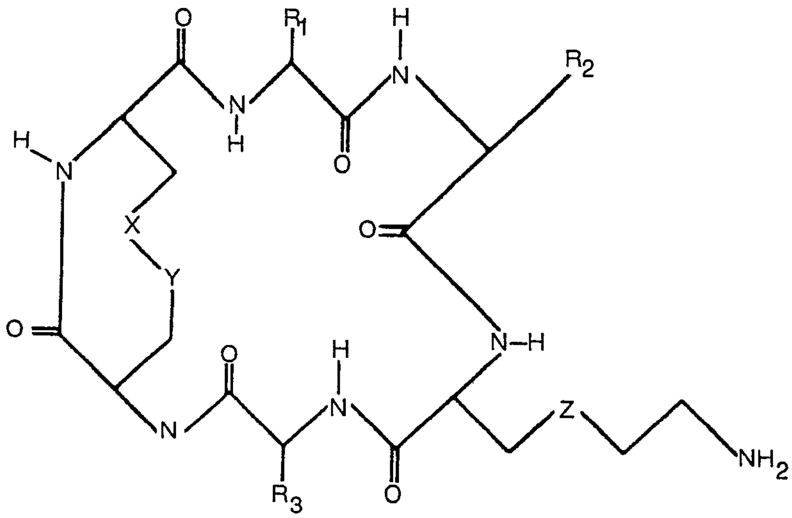

I

wherein

X and Y are S or $CH_2$ provided at least one of X and Y is S;

Z is $(CH_2)_m$, wherein m is 0, 1 or 2, or sulphur such that the sulfur may be in any position along the chain;

$R_1$ is a straight or branched chain alkyl group having 1—5 carbon atoms, benzyl, substituted benzyl wherein the substituent may be one or two of straight or branched chain alkyl groups having 1—5 carbon atoms, halogen, hydroxy, amino, nitro or straight or brnached chain alkoxy groups having 1—5 carbon atoms; and straight or branched chain alkyl having 1—5 carbon atoms which is substituted with a 5- or 6-membered heterocyclic ring;

$R_2$ is 3-indolylmethyl or substituted 3-indolylmethyl wherein the substituent may be straight or branched chain alkyl having 1—5 carbon atoms, straight or branched chain alkoxy having 1—5 carbon atoms or halogen;

$R_3$ is a straight or branched chain alkyl having 1—5 carbon atoms, hydroxy alkyl, wherein the alkyl group is straight or branched chain and comprises 1—5 carbon atoms, benzyl, carboxyalkyl wherein the alkyl group is straight or branched chain and comprises 1—5 carbon atoms, amino alkyl, wherein the alkyl group is straight or branched chain and comprises 1—5 carbon atoms, or substituted hydroxy-benzyl, wherein the substituent may be straight or branched chain alkyl having 1—5 carbon atoms, straight or branched chain alkoxy having 1—5 carbon atoms, hydroxy, halogen, amino or nitro,
or a salt of the compound of formula I.

The inventive dicyclic hexapeptides inhibit the release of glucagon, growth hormone and insulin and inhibit the release of gastric acid secretions, however, their inhibitory activities differ from the corresponding activities of somatostatin and accordingly said compounds have a higher biological selectivity than somatostatin.

Among the inventive compounds of formula I there are to be found such compounds which preferentially inhibit the release of growth hormone without affecting the level of gastric secretions alone or without affecting the level of gastric secretions, insulin and glucagon. There are, furthermore, to be found also such compounds which inhibit the release of gastric acid secretions.

A further object of the present invention are pharmaceutical compositions which comprise as active ingredient an inventive dicyclic hexapeptide of formula I or a non-toxic salt of the compounds of formula I.

Corresponding pharmaceutical preparations can be used for the treatment of acromegaly, diabetes, diabetic retinopathy and peptic ulcers.

In the inventive compounds of formula I examples for straight or branched alkyl groups having 1—5 carbon atoms are methyl, ethyl, propyl, *iso*-propyl, butyl, *sec*-butyl and pentyl.

Examples for straight or branched chain alkoxy groups having 1—5 carbon atoms are methoxy, ethoxy, propoxy, isopropoxy, butoxy, *tert*-butoxy and pentoxy.

The term "halogen" or "halo" is intended to include fluorine, chlorine, bromine and iodine.

Examples for radicals $R_1$ which are straight or branched chain alkyl radicals having 1—5 carbon atoms, which are substituted with a 5- or 6-membered heterocyclic ring, are such, which are substituted with corresponding heterocyclic rings comprising 1 or 2 heteroatoms selected from oxygen, nitrogen and sulfur. Examples of such heterocycles are imidazole, furan, thiazole, pyrazole and pyridine.

In the instant compounds there are several asymmetric centers which will lead to the existence of optical isomers for such compounds. In the instant invention, for each of the asymmetric centers of the various amino acids which make up the instant cyclic hexapeptides, both the D and L configurations are intended to be encompassed.

It will be appreciated by those skilled in the art that when $R_1$ is benzyl, $R_2$ is indolylmethyl, Z is methylene, and $R_3$ is 1-hydroxyethyl, the 7, 8, 9 and 10 amino acids of somatostatin (-Phe-Trp-Lys-Thr) are represented, and the Cys-Cys grouping with its bridged linkage has taken the place of the remainder of the somatostatin amino acids. Thus, using the above definitions of the substituent groups, the following representative cyclic hexapeptide analog of of somatostatin is formed in structure IC

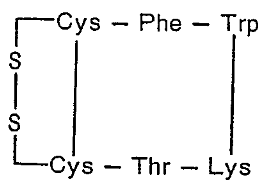

Ic.

Preferred examples of the inventive compounds of formula I are those in which
Z is the group —$CH_2$—;
$R_1$ is defined as in formula I;
$R_2$ is 3-indolylmethyl or substituted indolylmethyl wherein the substituent is methoxy or fluoro; and
$R_3$ is methyl, ethyl, hydroxymethyl or hydroxyethyl, or a salt of said preferred compounds of formula I.

Specially preferred of said group of inventive compounds of formula I are those wherein
Z is the group —$CH_2$—;
$R_1$ is defined as in formula I;
$R_2$ is 3-indolylmethyl; and
$R_3$ is hydroxyethyl,
or a salt of said specially preferred compounds of formula I.

In the inventive compounds of formula I the group
$R_1$ is preferably a straight or branched chain alkyl group having 1—5 carbon atoms, benzyl, substituted benzyl, wherein the substituent is a straight or branched chain alkyl group having 1—5 carbon atoms, halogen, hydroxy, amino, nitro or a straight or branched chain alkoxy group having 1—5 carbon atoms.

Included within these preferred disulfide bridged compounds are:

Cyclo-(Cys-Cys-Tyr-D-Trp-Lys-Thr)
Cyclo-(Cys-Cys-Tyr-D-Trp-Lys-Val)
Cyclo-(Cys-Cys-Tyr-L-Trp-Lys-Val)
Cyclo-(Cys-Cys-Phe-D-Trp-Lys-Thr)
Cyclo-(Cys-Cys-Phe-L-Trp-Lys-Thr)
Cyclo-(Cys-Cys-His-D-Trp-Lys-Thr)
Cyclo-(Cys-Cys-His-D-Trp-Lys-Val)

In the instant application several abbreviated designations are used for the amino acid components, certain preferred protecting groups, reagents and solvents. The meanings of such abbreviated designations are given in Table I.

TABLE I

| Abbreviated Designation | Amino Acid |
|---|---|
| Lys | L-lysine |
| Phe | L-phenylalanine |
| Trp | L-tryptophan |
| D-Trp | D-tryptophan |
| Thr | L-threonine |
| Aha | 7-aminoheptanoic acid |
| Tyr | L-tyrosine |
| Val | L-valine |
| Abu | L-α-aminobutyric acid |
| Ser | L-serine |
| Asn | L-asparagine |
| Pro | L-proline |
| Asu | D- or L-aminosuberic acid |
| Cys | L-cysteine |

| Abbreviated Designation | Protecting Groups |
|---|---|
| INOC | isonicotinyloxycarbonyl |
| BOC | tert-butyloxycarbonyl |
| OMe | methyl ester |
| Bu | tert-butyl |
| CBZ | benzyloxycarbonyl |
| Bzl | benzyl |
| 2-Cl-CBZ | 2-chlorobenzyloxycarbonyl |
| Acm | acetamidomethyl |
| Me | methyl |

| Abbreviated Designation | Activating Groups |
|---|---|
| ONp | p-nitrophenyl ester |
| HSE | N-hydroxysuccinimide ester |
| HBT | 1-hydroxybenzotriazole |

| Abbreviated Designation | Condensing Agents |
|---|---|
| DCCI | dicyclohexylcarbodiimide |

| Abbreviated Designation | Reagents |
|---|---|
| TFA | trifluoroacetic acid |
| TEA | triethylamine |
| DIPEA | diisopropylethylamine |

| Abbreviated Designation | Solvents |
|---|---|
| EPAW | ethyl acetate-pyridine-acetic acid-water |
| BAW | butanol-acetic acid-water |
| CMW | chloroform-methanol-water |
| DMF | dimethylformamide |
| THF | tetrahydrofuran |

A further object of the present invention are processes for preparing the inventive dicyclic hexapeptides of formula I.

One object is a process for preparing a disulfide bridge cyclic hexapeptide having the following formula Ia

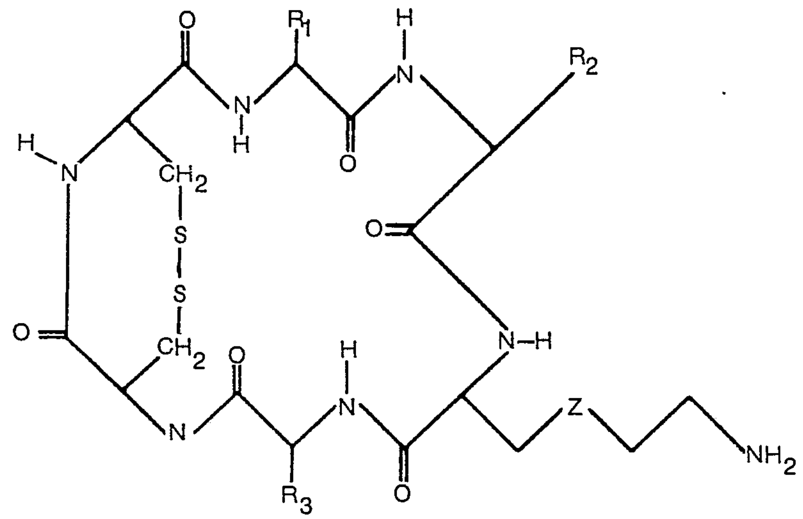

Ia

wherein

Z, $R_1$, $R_2$ and $R_3$ are as defined in formula I or a salt thereof. Said process is characterized in that the following reaction steps are performed:

a) preparing a corresponding blocked linear peptide attached to a solid phase resin;

b) selectively deblocking the N-terminal amine group;

c) removing the linear peptide from the resin;

d) treating the linear peptide with a cyclizing agent to form the amide bond of the desired cyclic hexapeptide;

e) removing the side chain protecting groups;

f) removing the cysteine protecting groups and creating the disulfide linkage and isolating the compound of formula Ia or a salt thereof.

When the linear peptide is prepared on the resin, it is generally not critical which amino acid is selected to be at the C-terminal position provided only that the sequence of amino acids in the linear peptide corresponds to that in the desired somatostatin analog. Once a linear peptide has been cyclized one can no longer determine which amino acid was at the C-terminus of the linear peptide.

While generally the selection of the first amino acid to start the chain is not critical, since the linear peptide will be cyclized, there may be other factors which may cause one to prefer one starting amino acid over another. For example D-Trp can react with t-butyl carbonium ions which are formed when BOC groups are removed. Thus, selection of a reaction sequence which places D-Trp at the N-terminal end of the linear peptide will cause D-Trp to be added last, and thus it will have the least exposure to t-butyl carbonium ions. This type of selection may not always be possible, such as where there are two indole containing moieties in the peptide. However, such reaction sensitivities should be considered when planning a peptide reaction sequence.

According to a preferred embodiment said process is performed as follows:

The synthesis of the linear peptides by the solid phase technique is conducted in a stepwise manner on chloromethylated resin. The resin is composed of fine beads (20—70 microns in diameter) of a synthetic resin prepared by copolymerization of styrene with 1—2 percent divinylbenzene. The benzene rings in the resin are chloromethylated in a Friedel-Crafts reaction with chloromethyl methyl ether and stannic chloride. The Friedel-Crafts reaction is continued until the resin contains 0.5 to 5 mmoles of chlorine per gram of resin.

The amino acid selected to be the C-terminal amino acid of the linear peptide is converted to its amino protected derivative. The carboxyl group of the selected C-terminal amino acid is bound covalently to the insoluble polymeric resin support, as for example, as the carboxylic ester of the resin-bonded benzyl chloride present in chloromethyl-substituted polystyrene-divinylbenzene resin. After the amino protecting group is removed, the amino protected derivatve of the next amino acid in the sequence is added along with a coupling agent, such as dicyclohexylcarbodiimide. The amino acid reactant may be employed in the form of a carboxyl-activated amino acid such as the ONp ester, an amino acid azide, and the like. Deprotection and addition of successive amino acids is performed until the desired linear peptide is formed.

The selection of protecting groups is, in part, dictated by particular coupling conditions, in part by the amino acid and peptide components involved in the reaction.

Amino-protecting groups ordinarily employed include those which are well known in the art, for example, urethane protecting substituents such as benzyloxycarbonyl (carbobenzoxy, p-

methoxycarbobenzoxy, p-nitrocarbobenzoxy, t-butyloxycarbonyl, and the like. It is preferred to utilize t-butyloxycarbonyl (BOC) for protecting the α-amino group in the amino acids undergoing reaction at the carboxyl end of said amino acid. The BOC protecting group is readily removed following such coupling reaction and prior to the subsequent step by the relatively mild action of acids (i.e. trifluoro acetic acid, or hydrogen chloride in ethyl acetate).

The sulfur of cysteine can be protected with the acetamidomethyl (Acm). A particular advantage of the Acm group is that it is not removed by treatment with HF and remains intact, to allow the isolation of the bridged cyclic hexapeptide in a sulfur-protected form at the penultimate stage. The Acm group is removed as part of the procedure leading to the disulfide bridged bicyclic final product. The second cyclization which prepares the bridging structure is accomplished with iodine in N,N-dimethylformamide or alternatively, by mercuric ion followed by oxidation.

The OH group of Thr and Ser can be protected by the Bzl group and the ε-amino group of Lys can be protected by the INOC group or the 2-chlorobenzyloxycarbonyl (2-Cl-CBZ) group. In the base of Lys, it is preferred to protect the ε-amino group with 2-Cl-CBZ group as this group is removed simultaneously with the Bzl groups by treatment with HF after the linear peptide has been cyclized. The INOC group is not removed by HF and requires an additional treatment with Zn. Neither group is affected by TFA, used for removing BOC protecting groups. After the linear peptide is cyclized, the protective groups, such as 2-Cl-CBZ and Bzl, are removed by treatment with HF.

After the linear peptide has been formed on the solid phase resin, it may be removed from the resin by a variety of methods which are well known in the art. For example, the peptide may be cleaved from the resin with hydrazide which may be subsequently cyclized via the azide to the desired cyclic peptide. The hydrazide is converted to the corresponding azide by reaction with a reagent which furnishes nitrous acid *in situ*. Suitable reagents for this purpose include a lower alkyl nitrite (e.g. t-butyl nitrite, isoamyl nitrite) or an alkali metal nitrite salt (e.g., sodium nitrite, potassium nitrite) in the presence of a strong acid such as hydrochloric, phosphoric, etc. This reaction is carried out in the presence of either water and/or a nonaqueous solvent such as dimethylformamide, tetrahydrofuran, dioxane, chloroform, methylene chloride, etc., at a temperature between about −40°C and +20°C. Alternatively, the peptide may be removed from the resin by treatment with a lower alcohol such as methanol in the presence of an organic base such as triethylamine, thus resulting in the formation of the corresponding lower alcohol ester of the linear peptide. The resulting ester may be converted to the hydrazide which may then be cyclized, via the azide, to the desired cyclic peptide. The preferred method for cleaving the peptide from the resin in the present invention is the use of hydrazine.

A further object of the present invention is a process for preparing a dicyclic hexapeptide having the followiing formula Ib

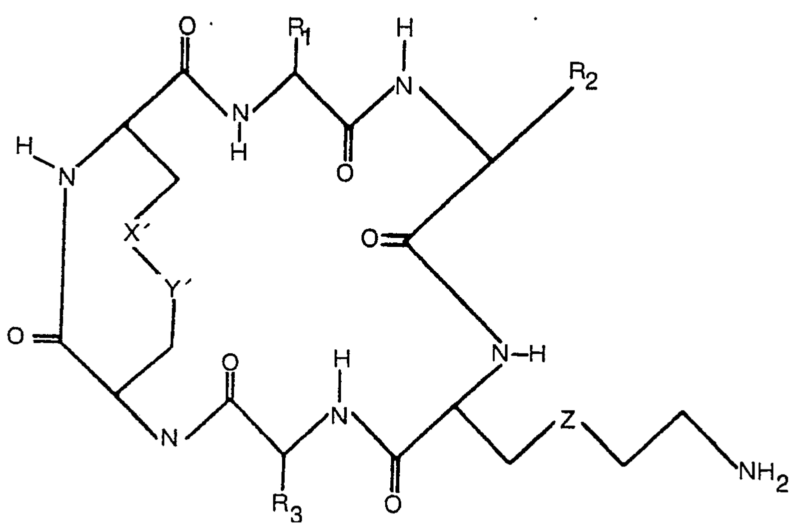

Ib

wherein
one of the groups X′ and Y′ is a sulfur atom while the other of said groups is a —$CH_2$-group and Z, $R_1$, $R_2$ and $R_3$ are as defined in formula I or a salt thereof. Said process is characterized in that

a) a corresponding blocked dicyclic peptide attached to the solid phase resin is prepared by using as one of the amino acid moieties in the solid phase synthesis a cyclic dipeptide having the structure IIa or IIb

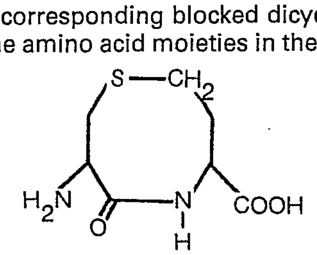

IIa

or

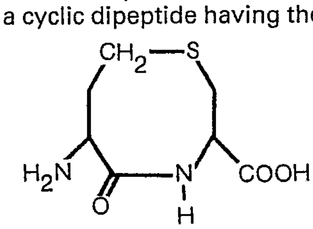

IIb

b) whereafter the N-terminal amino group of the monocyclic peptide bonded to the resin is removed,

c) the monocyclic peptide is removed from the resin,

d) the monocyclic peptide is treated with a cyclizing agent to form the amide bond of the desired dicyclic hexapeptide and

e) the side chain protecting groups are removed and the dicyclic peptide of formula Ib or a salt thereof is isolated.

According to said process the normally linear peptide which can be prepared by the solid phase technique is converted to a cyclic compound having the bridging group already in place. From the solid phase series of reactions the above cyclic dipeptides will have the other four amino acids substituted on the amino or carboxyl acid group or both. The distribution can be in any pattern with all of the other four amino acids on the amino, all on the carboxylic acid or distributed on both. When the monocyclic hexapeptide is cyclized the original order of the amino acids will be observed, as discussed above. In addition, since the bridging group is already in place, the final cyclization, used for the disulfide bridging group, is not necessary, and the bicyclic hexapeptide is formed directly.

Both monocyclic dipeptides II-a and II-b may be obtained from the same precursor, L-cystathionine, by direct cyclization using methods known in the art. The two possible dipeptide components are separated from any polymeric by-products using standard techniques, preferably, however, with gel filtration. They may be separated from one another by any of a variety of techniques known in the art, preferably silica gel chromatography.

The thus produced amino acid is protected at the amino group, preferably, by *t*-BOC, for incorporation into the linear hexapeptide sequence, in place of -Cys(Acm)-Cys(Acm)- using the solid phase technique. Removal from the solid support by hydrazinolysis, cyclization and blocking group removal affords the desired bicyclic product directly.

As reference Table II will show, one preferred overall procedure for preparing the desired disulfide bridged cyclic hexapeptides of the present involves the stepwise synthesis of the linear peptide on a solid phase resin. More specifically, in the process for preparing compound III:

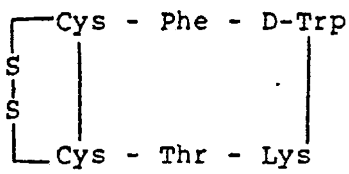

III

The carboxyl end of the N-blocked amino acid phenylalanine is bound covalently to an insoluble polymeric resin support as the carboxylic acid ester of the resin-bonded benzyl chloride. The amino group of Phe is protected by the BOC group. After the attachment of the Phe is completed on the resin, the protecting group BOC is removed by treatment with TFA in $CH_2Cl_2$. The subsequent amino acids are attached, in the form of BOC-amino acid, using DCCI as the condensing agent or an active ester such as ONp. After the desired linear peptide has been prepared, the N-terminal amino group is selectively deblocked and the peptide is removed from the resin by treatment with hydrazine. The resulting linear peptide hydrazide with the N-terminal amino group deblocked having the amino acid sequence:

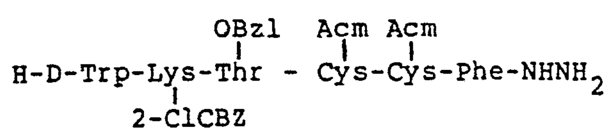

is treated with isoamyl nitrite in acid pH to form the corresponding azide. The azide solution is diluted with solvent and neutralized with an organic base. The linear peptide cyclizes to form:

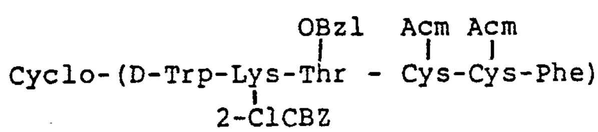

During the cyclization the pH is checked and maintained at neutral by the addition of organic base. The pH in organic solvent is determined by the application of an aliquot of the solution to moistened narrow range pH paper.

After the linear peptide is cyclized, the protective groups, 2-Cl-CBZ and OBzl, are removed by treatment with HF in the presence of anisole, and the Acm groups are removed and the disulfide bridge is simultaneously created by reacting the Acm-protected cyclic hexapeptide with iodine in N,N-dimethylformamide. The crude disulfide bridged cyclic peptide obtained is purified chromatographically, preferably by gel filtration. One example of such gel filtration is passing a solution to be purified through a column of Sephadex (a cross-linked dextran gel) and eluting with 50% acetic acid or with 2N-acetic acid. The elution of the desired product is detemrined by analyzing aliquots of the material using thin layer chromatography.

TABLE II

The reaction scheme for the preparation of one of the disulfide bridged cyclic hexapeptides of this invention is outlined in the following series of reactions:

Reaction scheme for preparing:

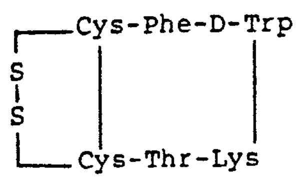

Cl-CH$_2$Ø-resin

BOC-Phe-OH

BOC-Phe-O-CH$_2$Ø-resin

Solid Phase Method

1) BOC-Cys(Acm)-OH
2) BOC-Cys(Acm)-OH
3) BOC-(OBzl)Thr-OH
4) BOC-( -2-Cl-CBZ)Lys-OH
5) BOC-D-Trp-OH

Acm Acm

BOC-D-Trp (ε-2-Cl-CBZ)Lys-(OBzl)Thr-Cys-Cys-Phe-OCH$_2$Ø-resin

TFA

Acm Acm

H-D-Trp-(ε-2-Cl-CBZ)Lys-(OBzl)Thr-Cys-Cys-Phe-O-CH$_2$Ø-resin

$NH_2NH_2$

Acm Acm

H-D-Trp-((-2-Cl-CBZ)Lys-(OBzl)Thr-Cys-Cys-Phe-NHNH$_2$

isoamyl nitrite, $H^+$, DMF

Acm Acm

H-D-Trp-((-2-Cl-CBZ)Lys-(OBzl)Thr-Cys-Cys-Phe-N$_3$

DMF/triethylamine

TABLE II (continued)

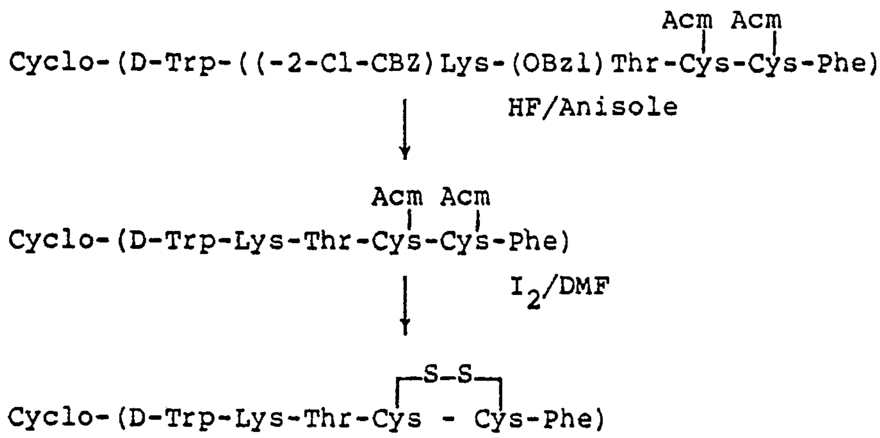


The present invention, furthermore, concerns monocyclic peptides used as starting materials for performing said process.

A further object of the present invention accordingly are monocyclic dipeptides having the formulae IIa or IIb

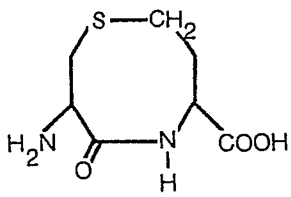

IIa

or

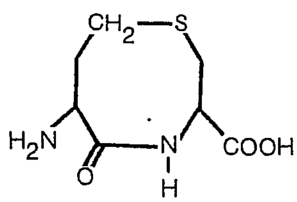

IIb

and protected derivatives of the monocyclic dipeptides of formula IIa and IIb.

Methods for preparing the inventive dicyclic hexapeptides and the starting materials used in said process are illustrated with the following not limitative examples.

Example 1

Preparation of H-D-Trp-(ε-2-Cl-CBZ)Lys-(OBzl)Thr-Cys(Acm)-Cys(Acm)-Phe-$OCH_2$Ø-resin

Chloromethyl resin (2% cross-linked Merrifield resin), 862.0 g (2.37 moles), having 2.75 meq. chlorine/g, and 607.0 g (2.37 moles, 1 equivalent) of BOC-Phe-OH were added to 4320 ml of peroxide-free tetrahydrofuran. The mixture was stirred in an oil bath at 80°C bath temperature for 45 minutes. Triethylamine, 310.0 ml, was added and the reaction mixture stirred at 80°C bath temperature for 70 hours, cooled to 25°C and transferred to a stirred solid phase reaction column with 2000 ml of tetrahydrofuran. After removal of the solvent, the resin was washed using the stirred column with:

3 × 2000 ml of tetrahydrofuran
4 × 5170 ml of ethanol
1 × 5170 ml of acetic acid
3 × 5170 ml of water
3 × 5170 ml of methanol
3 × 5170 ml of chloroform

The BOC-Phe-O-$CH_2$Ø-resin was dried *in vacuo* at 25°C for 16 hours, giving 1203 g of BOC-O-$CH_2$Ø-resin containing 1.2 mmole of phenylalanine/g of resin.

BOC-Phe-O-$CH_2$Ø-resin (1.65 g; 2.0 mmole) was carried through the procedures in Tables III and IV using 2 deblockings (2 minutes and 25 minutes) with 25% TFA in methylene chloride and 2.5 equivalents of BOC-amino acid in the required sequence until the desired BOC-hexapeptide-O-$CH_2$Ø-resin was obtained.

DCCI was used as the sole coupling agent in every step.

The coupling of each amino acid proceeded smoothly. Best yields were obtained when the coupling was repeated in each step. When the coupling was repeated, the initial two chloroform washes were all omitted and replaced by a single chloroform wash.

The coupling reactions were carried out in methylene chloride, freshly degassed DMF or a mixture of these two solvents. The N-terminal amino group was blocked with a BOC group in each case; the hydroxy group of Thr was blocked with Bzl and the ε-amino group of Lys with 2-Cl-CBZ.

When the desired BOC-hexapeptide-O-$CH_2$Ø-resin was obtained, the N-terminal BOC group was removed by the terminal deblocking procedure set forth in Table V.

TABLE III

| Solvent or reagent (number of treatments or washes) | $CHCl_3$ (2) | 25% TFA in $CH_2Cl_2$ | $CHCl_3$(3) | $NEt_3$-$CH_2Cl_2$ (1:9) (2) | $CHCL_3$(3) $CH_2Cl_2$(3) | BOC AA in $CH_2Cl_2$ DMF or a mixture of both | 0.5 M DCCI in $CH_2Cl_2$ | DMF(1) MeOH(1) DMF(1) MeOH(1) $CHCl_3$(2) |
|---|---|---|---|---|---|---|---|---|
| Vol. in ml | 40 | 20 | 40 | 40 | 40 | 25 | 10 | 40 |
| Time in min. | 5 | 2 and 25 | 2 | 5 and 5 | 2 | 5 | 5 coupling 30 | 2 |

TABLE IV

| Protected Amino Acid | Solvent M1 |
|---|---|
| BOC-Cys(Acm)<br>Recouple | 20 ml $CH_2Cl_2$ |
| BOC-Cys(Acm)<br>Recouple | 20 ml $CH_2Cl_2$ |
| BOC (OBzl)Thr (1.55 g)<br>Recouple | 20 ml $CH_2Cl_2$ |
| BOC-(ε 2-Cl-CBZ)Lys (2.24 g)<br>Recouple | 20 ml $CH_2Cl_2$ |
| BOC-D-Trp (1.52 g)<br>Recouple | 15 ml $CH_2Cl_2$, 5 ml DMF |

TABLE V
TERMINAL DEBLOCKING PROGRAM

| Solvent or reagent Number of treatments or washes) | $CHCl_3$(1) | 25% TFA in $CH_2Cl_2$ + 1% Ethanedithiol (2) | $CHCl_3$ (3) | MeOH(2) $CH_2Cl_2$(1) MeOH(2) $CH_2Cl_2$(2) |
|---|---|---|---|---|
| Vol. in ml. | 40 | 40 | 40 | 40 |
| Time in minutes | 5 | 2 and 25 | 2 | 2 |

After the procedures of Tables III, IV and V were completed, the blocked hexapeptide-$OCH_2\emptyset$-resin is dried overnight and weighs 4.00 g.

Example 2

Preparation of D-Trp-(ε-2-Cl-CBZ)Lys-(OBzl)Thr-Cys(Acm)-Cys(Acm)-Phe-$NHNH_2$

The resin from Example 1 was combined with 36 ml of a 9:1 mixture of N,N-dimethylformamide and hydrazine and stirred at room temperature for 1 hour. The insoluble resin was removed by filtration and the solution was evaporated to remove the N,N-dimethylformamide. The addition of 50 ml of water followed by trituration gave a solid. The solid was isolated by filtration, and washed thoroughly with water. The solid was dried *in vacuo* and weighed 246 g.

Example 3

Preparation of H-D-Trp-(ε-2-Cl-CBZ)Lys-(OBzl)Thr-Cys(Acm)-Cys(Acm)-Phe-$N_3$

2,41 g of the product from Example 2 was combined with 40 ml of degassed N,N-dimethylformamide under a blanket of nitrogen, cooled to −10°C, and 5 equivalents of 5.9M hydrogen chloride in 2.0 ml of tetrahydrofuran was added. The solution was cooled to −25°C and 0.31 ml of a isoamyl nitrite was added in portions until a positive starch/KI test was obtained. The completion of the reaction was determined by thin layer chromatography and the disappearance of the hydrazide starting material.

Example 4

Preparation of Cyclo(D-Trp-(ε-2-Cl-CBZ)Lys-(O-Bzl)Thr-Cys(Acm)-Cys(Acm)-Phe)

The solution of the azide compound of Example 3 was added to 1.2 l of degassed dimethylformamide, precooled to −25°C, the pH adjusted to 8, with diisopropylethylamine, and the reaction mixture placed in the freezer (−25°C) overnight. The pH was readjusted to 8 if necessary after about 14 hours and the mixture stored for 16 hours at −20°C and 16 hours at 5°C. Thin layer chromatography indicated that the reaction was complete. The mixture was concentrated to dryness, and the residue was triturated with 50 ml of water to give a white solid. The solid was isolated by filtration, washed with water and dried *in vacuo*. The product weighed 2.30 g.

Example 5

Preparation of Cyclo(D-Trp-Lys-Thr-Cys(Acm)-Cys(Acm)-Phe)

2.03 g (1.7 mmoles) of the protected cyclic hexapeptide of Example 4 was combined in a teflon lined chamber with 2 ml of anisole. The chamber was then evacuated and filled with 20 ml of liquid hydrogen fluoride at the temperature of the dry ice/acetone bath. The temperature was raised to 0°C and stirred for 1 hour. The hydrogen fluoride was allowed to evaporate and the residue placed *in vacuo* until a slurry was formed. The slurry was treated with 50 ml of ethyl acetate and filtered affording 1.87 g of a fine powder. The crude product was dissolved in 50% acetic acid, placed on a column of Sephadex® G-25 gel (superfine) and eluted with the same solvent. The presence of the product in the eluent was determined by thin layer chromatographic analysis. The residue after evaporation of the pooled fractions and freeze drying, afforded 1.17 g of product.

Example 6

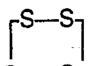

Preparation of Cyclo(D-Trp-Lys-Thr-Cys -Cys-Phe)

254 Mg of iodine (1.0 mmole) was dissolved in 90 ml of freshly degassed DMF and added to a solution of 182 mg (0.2 mmole) of the product of Example 5 dissolved in 90 ml of freshly degassed DMF. The mixture was stirred for 3½ minutes and 600 mg of zinc dust and 40 ml of 50% aqueous acetic acid at 0°C was added with stirring. The temperature spontaneously rose to 30°C. The reaction was decolorized with carbon, filtered and the filtrate washed twice with DMF. The solution was evaporated to dryness, dissolved in 50% aqueous acetic acid and placed on a column of Sephadex® G-25 gel (superfine) eluted with the same solvent affording 66.9 mg of product.

The sample thus isolated was found to be unstable with respect to polymerization in certain solvents or on extended storage. In order to stabilize the compound toward such decomposition it was treated in aqueous solution with 10—40 mole % of N-ethylmaleimide, a reagent which will react with free sulfhydryl groups that could catalyze disulfide interchange. The excess N-ethyl maleimide was separated by passage through a column of Sephadex® G-25 gel (superfine) eluted with 50% acetic acid. The peptide was reisolated by freeze-drying of the pooled column fractions after evaporation and shown to be completely stable.

Following the above procedure, either Examples 1—6 or 1—5 and 7 and by modifying only the selection and order of amino acids in the process of Example 1, there are prepared other cyclic hexapeptides of this invention.

The instant cyclic hexapeptide analogs of somatostatin are tested and compared with the effects of somatostatin in an *in vitro* test for the inhibition of growth hormone. The test is described as follows:

"Rat pituitaries were isolated according to the procedures of Vale and Grant 'In Vitro Pituitary Hormone Secretion Assay for Hypophysiotropic Substances' in Methods in Enzymology. Vol. XXXVII, eds. O'Malley, B. W. and Hardman, J. G. (Academic Press, Inc., New York) pp. 5—93 (1975).

After 4 days in culture, the cells were washed and incubated for 4 hours in Dulbecco-modified Eagle's medium in the presence or absence of graded doses of each analog or somatostatin. The medium was then collected for subsequent growth hormone determination by a double antibody radioimmunoassay for rat growth hormone."

Example 7

Preparation of

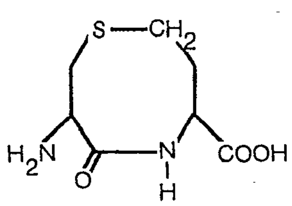

and

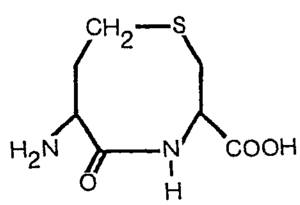

A solution of 222 mg of L-cystathionine in 500 ml of DMF at 0° is treated with TEA to give a pH of 7.5 (as determined by moistened narrow-range pH paper), followed by 0.24 ml of diphenylphosphoryl azide (DPPA). Stirring is maintained and TEA is added periodically to maintain pH 7.5. Completion of the reaction is determined by thin layer chromatography and the disappearance of the starting material. The solvent is removed by evaporation, and the residue is dissolved in 50% acetic acid and placed onto a column of Sephadex G-25 gel. Elution is accomplished with the same solvent to afford a mixture of the two desired monocyclic monomeric products. These components are separated by silica gel or ion exchange chromatography, and isolated independently by freeze-drying.

The compounds thus isolated may be suitably protected and employed as one of the amino acids reacted with the resin-bound peptide in Example 1 and caried through to Example 5. The cyclization process of Example 6 is not employed since the thiomethylene bridge analog to the disulfide bridge is prepared above in an early step in the synthesis and not reserved for the end of the synthesis as is the formation of the disulfide bridge.

Analogs of somatostatin were compared to somatostatin in their ability to decrease the levels of portal vein glucagon and insulin in anesthetized rats. Male Sprague-Dawley rats (Charles River CD) weighing 160—200 g were anesthetized with urethane (150 mg/100 g of body weight; Aldrich). Saline or peptides were administered via the external jugular vein. After 5 minutes, the portal vein was exposed, and blood was collected via syringe containing 3 mg of EDTA and placed in chilled tubes containing 100 µl of Trasylol (FBA Pharmaceuticals) for subsequent hormone analysis. Plasma levels of glucagon were determined by the method of Faloona and Unger, *Methods of Hormone Radioimmunoassay*, Jaffe and Behrman (Eds), Academic Pess, New York, Vol. II, pp. 257—527 (1976), utilizing glucagon antisera 30K obtained from R. Unger (Dallas, TX). Plasma of insulin were determined by a modification of the procedure of Herbert *et al., J. Clin. Endcrinol. Metab., 25*, 1375-1384 (1965).

The test results for some of the compounds of this invention are recorded below with the results for somatostatin listed first and given the arbitrary value of 1. The results for the instant compounds are given as multiples or fractions of the effect of somatostatin. The numbers in parentheses are the fiducial limits for the number preceding. The first of the instant compounds listed is the compound prepared in Examples 1—5. The compound is written slightly different, however, to conform to the order of the amino acids found in somatostatin.

Activity of Cyclichexapeptide analogs of Somatostatin

| Compound | Growth Hormone Release Inhibition | Insulin Inhibition | Glucagon Inhibition |
|---|---|---|---|
| Somatostatin | 1 | 1 | 1 |
| Cyclo(Cys-Cys-Phe-D-Trp-Lys-Thr) | 2.47 (1.66—3.81) | 10.6 (5.5—19.9) | Active |

**Claims**

1. A compound having the formula I

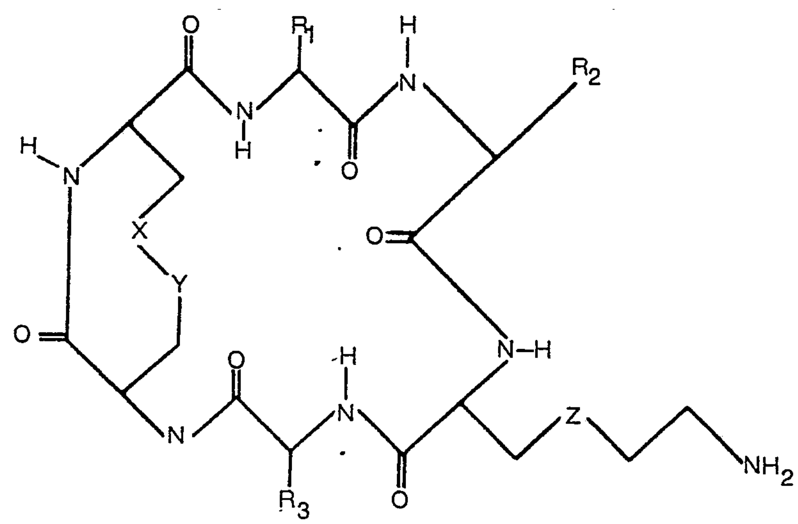

I

wherein

X and Y are S or $CH_2$ provided at least one of X and Y is S;

Z is $(CH_2)_m$ wherein m is 0, 1 or 2 or sulphur such that the sulfur may be in any position along the chain;

$R_1$ is a straight or branched chain alkyl group having 1—5 carbon atoms, benzyl, substituted benzyl wherein the substituent may be one or two of straight or branched chain alkyl groups having 1—5 carbon atoms, halogen, hydroxy, amino, nitro or straight or branched chain alkoxy groups having 1—5 carbon atoms; and straight or branched chain alkyl having 1—5 carbon atoms which is substituted with a 5- or 6-membered heterocyclic ring.

$R_2$ is 3-indolylmethyl or substituted 3-indolymethyl wherein the substituent may be straight or branched chain alkyl having 1—5 carbon atoms, straight or branched chain alkoxy having 1—5 carbon atoms or halogen;

$R_3$ is straight or branched chain alkyl having 1—5 carbon atoms, hydroxy alkyl, wherein the alkyl group is straight or branched chain and comprises 1—5 carbon atoms, benzyl, carboxyalkyl wherein the alkyl group is straight or branched chain and comprises 1—5 carbon atoms, amino alkyl, wherein the alkyl group is straight or branched chain and comprises 1—5 carbon atoms, or substituted hydroxy-benzyl, wherein the substituent may be straight or branched chain alkoxy having 1—5 carbon atoms, straight or branched chain alkoxy having 1—5 carbon atoms, hydroxy, halogen, amino or nitro,

or a salt of the compound of formula I.

2. A compound according to claim 1, characterized in that in formula I

Z is the group $—CH_2—$;

$R_1$ is defined as in claim 1;

$R_2$ is 3-indolylmethyl or substituted indolylmethyl wherein the substituent is methoxy or fluoro; and

$R_3$ is methyl, ethyl, hydroxymethyl or hydroxymethyl,

or a salt thereof.

3. A compound according to claim 1 or 2, characterized in that in formula I

Z is the group $—CH_2—$;

$R_1$ is defined as in claim 1;

$R_2$ is 3-indolylmethyl; and

$R_3$ is hydroxyethyl,

or a salt thereof.

4. A compound according to claim 1 or 2, characterized in that it is the

Cyclo-(Tyr-D-Trp-Lys-Thr-Cys-Cys)
S-S

or a salt thereof.

5. A compound according to claim 1 or 2, characterized in that it is the

Cyclo-(Phe-D-Trp-Lys-Thr-Cys-Cys)
S-S

or a salt thereof.

6. A process for preparing a disulfide bridged cyclic hexapeptide compound of claim 1 having the formula Ia

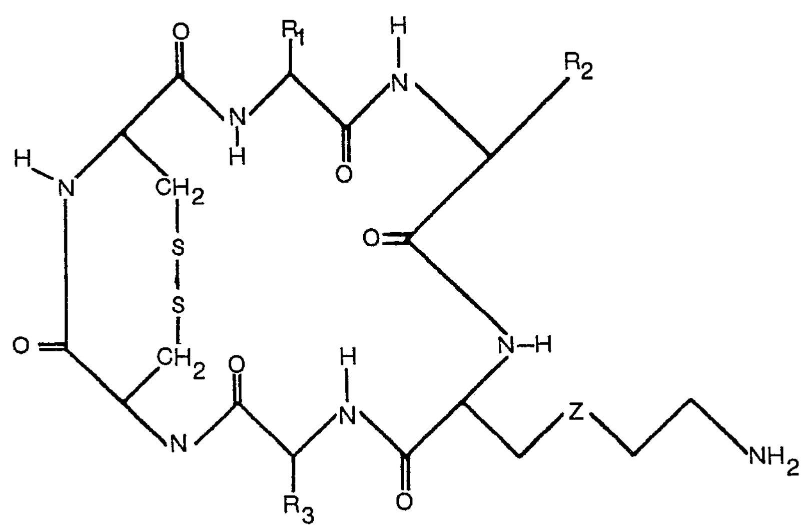

Ia

wherein

Z, $R_1$, $R_2$ and $R_3$ are as defined in claim 1 or a salt thereof, characterized in that the following reaction steps are performed;

a) preparing a corresponding blocked linear peptide attached to a solid phase resin;

b) selectively deblocking the N-terminal amine group;

c) removing the linear peptide from the resin;

d) treating the linear peptide with a cyclizing agent to form the amide bond of the desired cyclic hexapeptide;

e) removing the side chain protecting groups;

f) removing the cysteine protecting groups and creating the disulfide linkage and isolating the compound of formula Ia or a salt thereof.

7. Process according to claim 6 wherein step c) comprises treating the linear peptide resin with hydrazine to form the hydrazide.

8. Process for preparing a dicyclic hexapeptide of claim 1 having the formula Ib

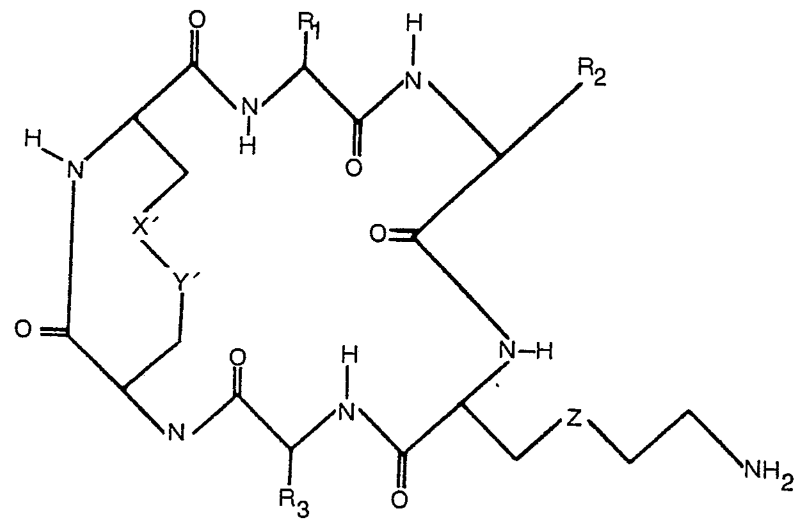

Ib

wherein one of the groups X′ and Y′ is a sulfur atom while the other of said group is a $-CH_2-$ group and Z, $R_1$, $R_2$ and $R_3$ are as defined in claim 1 or a salt thereof, characterized in that

a) a corresponding blocked dicyclic peptide attached to the solid phase resin is prepared by using as one of the amino acid moieties in the solid phase synthesis a cyclic dipeptide having the structure IIa or IIb

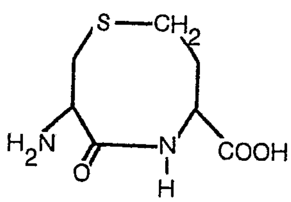

IIa

or

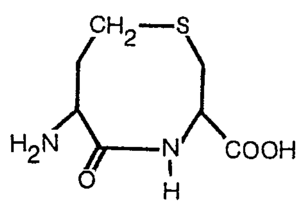

IIb

b) whereafter the N-terminal amino group of the monocyclic peptide bonded to the resin is removed, thereafter

c) the monocyclic peptide is removed from the resin,

d) the monocyclic peptide is treated with a cyclizing agent to form the amide bond of the desired dicyclic hexapeptide and

e) the side chain protecting groups are removed and the dicyclic peptide of formula Ib or a salt thereof is isolated.

9. A monocyclic dipeptide having the formulae IIa or IIb

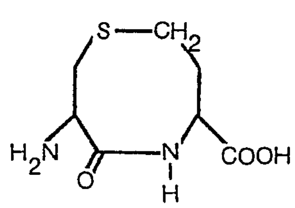

IIa

or

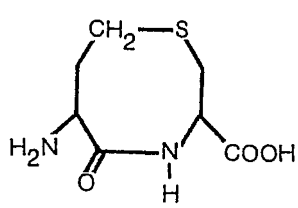

IIb

and protected derivatives of the monocyclic dipeptide of formulae IIa and IIb.

10. Pharmaceutical composition characterized in that it comprises as active ingredient a dicyclic hexapeptide of formula I according to claim 1 or a nontoxic salt of the compound of formula I.

**Patentansprüche**

1. Verbindung mit der Formel I

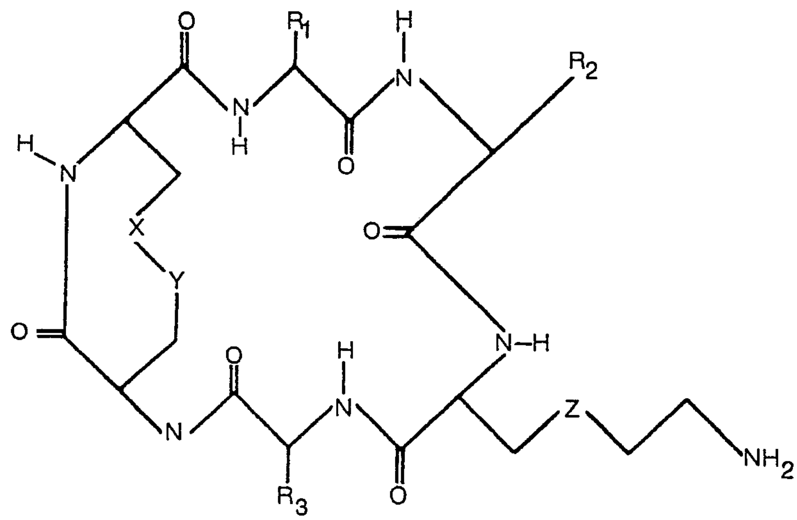

I

worin

X und Y S oder $CH_2$ sind, mit der Maßgabe, daß wenigstens eines aus X und Y S ist;

Z $(CH_2)_m$, worin m 0, 1 oder 2 ist, oder Schwefel ist, so daß der Schwefel jede Stellung in der Kette einnehmen kann;

$R_1$ eine geradkettige oder verzweigte Alkylgruppe mnit 1 bis 5 Kohlenstoffatomen, Benzyl, substituiertes Benzyl, worin der Substituent eines oder zwei aus geradkettigen oder verzweigen Alkylgruppen mit 1 bis 5 Kohlenstoffatomen, Halgen, Hydroxy, Amino, Nitro oder geradkettigen oder verzweigten Alkoxygruppen mit 1 bis 5 Kohlenstoffatomen sein kann; und geradkettiges oder verzweigtes Alkyl mit 1 bis 5 Kohlenstoffatomen, substituiert mit einem 5- oder 6-gliedrigen heterocyclischen Ring, ist;

$R_2$ 3-Indolylmethyl oder substituiertes 3-Indolylmethyl ist, worin der Substituent geradkettiges oder verzweigtes Alkyl mit 1 bis 5 Kohlenstoffatomen, geradkettiges oder verzweigtes Alkoxy mit 1 bis 5 Kohlenstoffatomen oder Halogen sein kann;

$R_3$ geradkettiges oder verzweigtes Alkyl mit 1 bis 5 Kohlenstoffatomen, Hydroxyalkyl, worin die Alkylgruppe geradkettig oder verzweigt ist und 1 bis 5 Kohlenstoffatome umfaßt, Benzyl, Carboxyalkyl, worin die Alkylgruppe geradkettig oder verzweigt ist, und 1 bis 5 Kohlenstoffatome umfaßt, Aminoalkyl, worin die Alkylgruppe geradkettig oder verzweigt ist und 1 bis 5 Kohlenstoffatome umfaßt, oder substituiertes Hydroxybenzyl, worin der Substituent geradkettiges oder verzweigtes Alkyl mit 1 bis 5 Kohlenstoffatomen, geradkettiges oder verzweigtes Alkoxy mit 1 bis 5 Kohlenstoffatomen, Hydroxy, Halogen, Amino oder Nitro sein kann ist,

oder ein Salz der Verbindung der Formel I.

2. Verbindung nach Anspruch 1, dadurch gekennzeichnet, daß in Formel I

Z die Gruppe —$CH_2$— ist;

$R_1$ wie in Anspruch 1 definiert ist;

$R_2$ 3-Indolymethyl oder substituiertes Indolymetyl ist; worin der Substituent Methoxy oder Fluor ist; und

$R_3$ Methyl, Ethyl, Hydroxymethyl oder Hydroxyethyl ist;

oder ein Salz davon.

3. Verbindung nach Anspruch 1 oder 2 dadurch gekennzeichnet, daß in Formel I

Z die Gruppe —$CH_2$— ist;

$R_1$ wie in Anspruch 1 definiert ist;

$R_2$ 3-indolylmethyl ist; und

$R_3$ Hydroxyethyl ist;

oder ein Salz davon.

4. Verbindung nach Anspurch 1 oder 2, dadurch gekennzeichnet, daß sie das

Cyclo-(Tyr-D-Trp-Lys-Thr-Cys-Cys)
S-S

oder ein Salz davon ist.

5. Verbindung nach Anspruch 1 oder 2, dadurch gekennzeichnet, daß sie das

Cyclo-(Phe-D-Trp-Lys-Thr-Cys-Cys)
S-S

oder ein Salz davon ist.

6. Verfahren zur Herstellung einer disulfid-verbrückten cyclischen Hexapeptidverbindung nach Anspruch 1 mit der Formel Ia

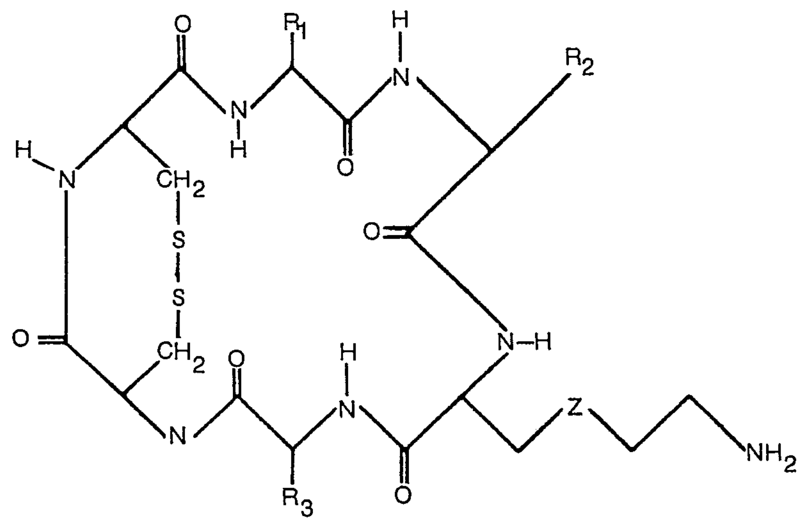

Ia

worin Z, $R_1$, $R_2$ und $R_3$ wie in Anspruch 1 definiert sind, oder eines Salzes davon, dadurch gekennzeichnet, daß die nachstehenden Reaktionsschritte durchgeführt werden:

a) Herstellung eines entsprechenden blockierten linearen Peptids, das an ein in fester Phase vorliegendes Harz gebunden ist;

b) selektives Deblockieren der N-terminalen Aminogruppe;

c) Entfernen des linearen Peptids von dem Harz;

d) Behandeln des linearen Peptids mit einem Cyclisierungsmittel unter Bildung der Amidbindung des gewünschten cyclischen Hexapeptids;

e) Entfernen der Seitenkettenschutzgruppe;

f) Entfernen der Cysteinschutzgruppe und Ausbilden der Disulfidbindung sowie Isolieren der Verbindung der Formel Ia oder eines Sazles davon.

7. Verfahren nach Anspruch 6, worin Schritt c) die Behandlung des linearen Peptidharzes mit Hydrazin unter Bildung des Hydrazids umfaßt.

8. Verfahren zur Herstellung eines dicyclischen Hexapeptids nach Anspurch 1 mit der Formel Ib

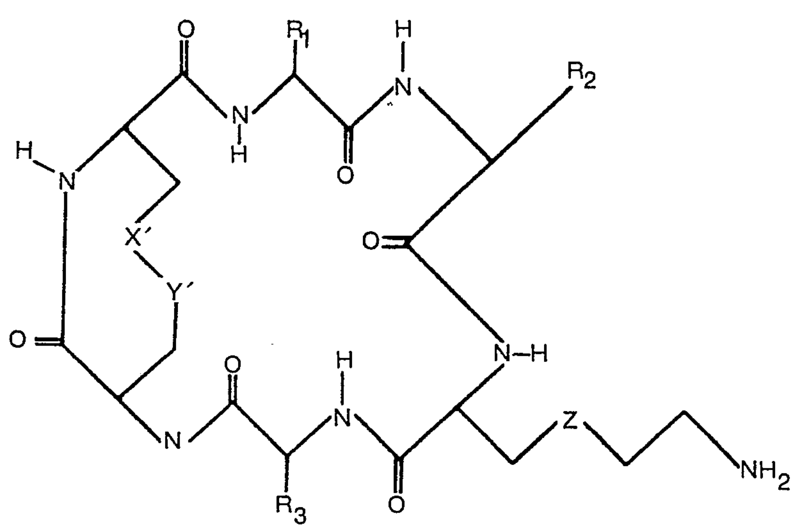

Ib

worin eine der Gruppen X' und Y' ein Schwefelatom ist, während die andere dieser Gruppen eine $-CH_2$-Gruppe ist und Z, $R_1$, $R_2$ und $R_3$ wie in Anspruch 1 definiert sind, oder eines Salzes davon, dadurch gekennzeichnet, daß

a) ein entsprechend blockiertes dicyclisches Peptid, das an ein in der festen Phase vorliegendes Harz gebunden ist, unter Verbindung eines cyclischen Dipeptids mit der Struktur IIa oder IIb

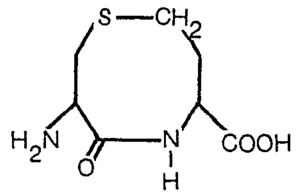
IIa

oder

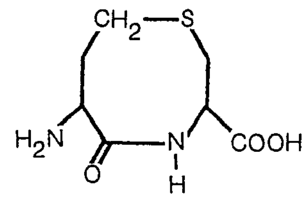
IIb

als einer der Aminosäureeinheiten in der Festphasensynthese hergestellt wird,

b) wonach die N-terminale Aminogruppe des an das Harz gebundenen monocyclischen Peptids entfernt wird, wonach

c) das monocyclische Peptid vom Harz entfernt wird,

d) das monocyclische Peptid mit einem Cyclisierungsmittel unter Bildung der Amidbindung des erwünschten dicyclischen Hexapeptids behandelt wird und

e) die Seitenketten-Schutzgruppen entfernt werden sowie das dicyclische Peptid der Formel Ib oder eine Salz isoliert wird.

9. Monocyclisches Dipeptid mit den Formeln IIa oder IIb

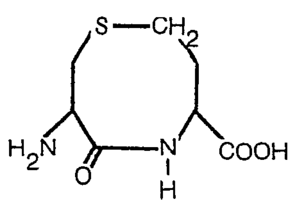
IIa

oder

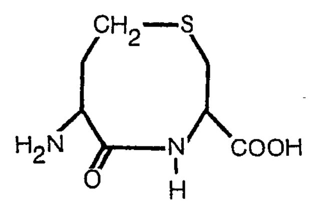
IIb

sowie geschützte Derivate des monocyclischen Dipeptids der Formeln IIA und IIB.

10. Pharmazeutische Zusammensetzung, dadurch gekennzeichnet, daß sie als aktiven Bestandteil ein dicyclisches Hexapeptid der Formel I nach Anspruch 1 oder ein nicht-toxisches Salz der Verbindung der Formel I umfaßt.

**Revendications**

1. Composé de formule I

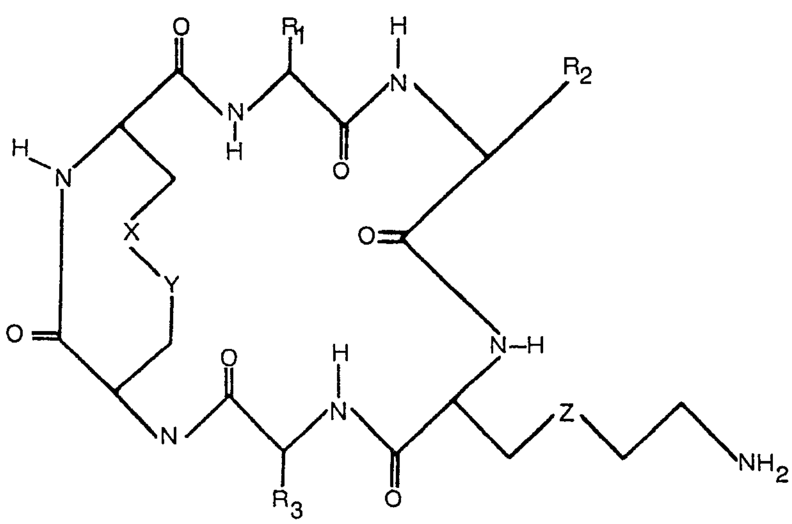
I

dans laquelle

X et Y sonts S ou $CH_2$, à condition que l'un au moins de X et de Y soit S;

Z est $(CH_2)_m$, où n est égal à 0, 1 ou 2, ou un soufre tel que le soufre puisse se trouver en toute position le long de la chaîne.

$R_1$ est un groupe alkyle à chaîne droite ou ramifiée comportant 1 à 5 atomes de carbone, un groupe benzyle, benzyle substitué dans lequel le substituant peut être un ou deux groupes alkyle à chaîne droite ou ramifiée comportant 1 à 5 atomes de carbone, des groupes halogéno, hydroxy, amino, nitro ou alcoxy à chaîne droite ou ramifiée comportant 1 à 5 atomes de carbone; et un groupe alkyle à chaîne droite ou ramifiée comportant 1 à 5 atomes de carbone, substituant par un noyau hétérocyclique à 5 ou 6 chaînons;

$R_2$ est un groupe 3-indolyméthyle ou 3-indolylméthyle substitué, dans lequel le substitué peut être un groupe alkyle à chaîne droite ou ramifiée comportant 1 à 5 atomes de carbone, un groupe alcoxy à chaîne droite ou ramifiée comportant 1 à 5 atomes de carbone ou un halogène;

$R_3$ est un groupe alkyle à chaîne droite ou ramifiée comportant 1 à 5 atomes de carbone, un groupe hydroxylalkyle, dans lequel le groupe alkyle est à chaîne droite ou ramifiée et comprend 1 à 5 atomes de carbone, un groupe benzyle, carboxyalkyle dans lequel le groupe alkyle est à chaîne droite ou ramifiée et comprend 1 à 5 atomes de carbone, un groupe aminoalkyle dans lequel le groupe alkyle est à chaîne droite ou ramifiée et comprend 1 à 5 atomes de carbone, ou un groupe hydroxybenzyle subtitué dans lequel le substituant peut être un groupe alkyle à chaîne droite ou ramifiée comportant 1 à 5 atomes de carbone, un groupe alcoxy à chaîne droite ou ramifiée comportant 1 à 5 atomes de carbone, un groupe hydroxy, halogéno, amino ou nitro,

ou un sel du composé de formule I.

2. Composé selon la revendication 1, caractérisé en ce que, dans la formule I,

Z est le groupe $—CH_2—$;

$R_1$ est défini comme dans la revendication 1;

$R_2$ est un groupe 3-indolylméthyle ou indolylméthyle substitué dans lequel le substituant est un méthoxy ou un flour; et

$R_3$ est un groupe méthyle, éthyle, hydroxyméthyle ou hydroxyéthyle,

ou un de ses sels.

3. Composé selon la revendication 1 ou 2, caractérisé en ce que, dans la formule I,

Z est le groupe $—CH_2—$;

$R_1$ est tel que défini dans la revendication 1;

$R_2$ est un group 3-indolylméthyle; et

$R_3$ est un groupe hydroxyéthyle,

ou un des ses sels.

4. Composé selon la revendication 1 ou 2, caractérisé en ce qu'il s'agit du

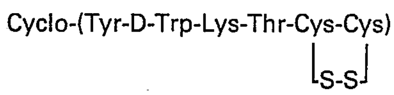

ou d'un de ses sels.

5. Composé selon la revendication 1 ou 2, caractérisé en ce qu'il s'agit du

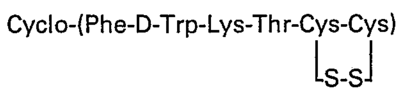

ou d'un ses sels.

6. Procédé de préparation d'un composé hexapeptide cyclique à pont disulfure selon la revendication 1, répondant à la formule Ia

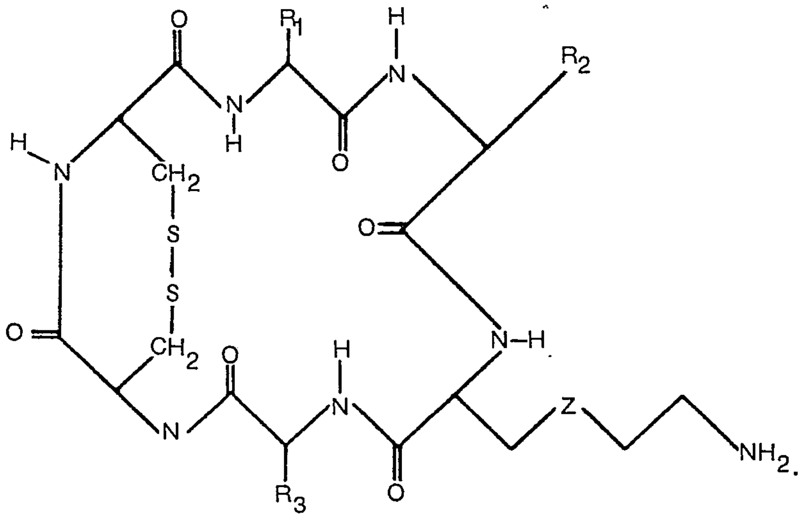

Ia

dans laquelle

Z, $R_1$, $R_2$ et $R_3$ sont tels que définis dans la revendication 1, ou d'un de ses sels, caractérisé en ce que l'on effectue les étapes réactonnelles suivantes:

a) préparation d'un peptide linéaire bloqué correspondant, fixé à une résine en phase solide;

b) déblocage sélectif du groupe amine N-terminal;

c) élimination du peptide linéaire de la résine;

d) traitement du peptide linéaire avec un agent de cyclisation pour former la liaison amide de l'hexapeptide cyclique recherché;

e) élimination des groupes protecteurs des chaînes latérales;

f) élimination des groupes protecteurs de cystéine et création de la liasion disulfure, et isolement du composé de formule Ia ou d'un ses sels.

7. Procédé selon la revendication 6, dans lequel l'étape c) comprend le traitement de la résine peptidique linéaire avec de l'hydrazine pour former l'hydrazide.

8. Procédé de préparation d'un hexapeptide dicyclique selon la revendication 1, répondant à la formule Ib

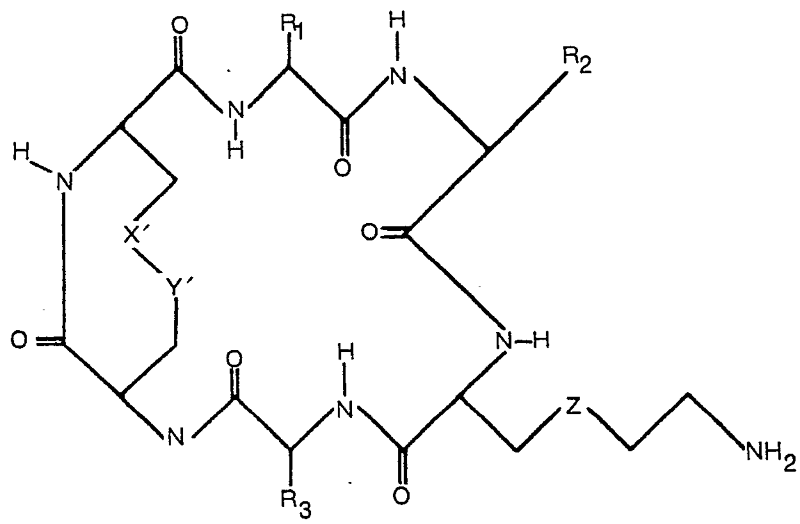

Ib

dans laquelle

l'un des groupes X' et Y' est un atome de soufre, tandis que l'autre de ces groupes est un groupe $-CH_2-$, et

Z, $R_1$, $R_2$ et $R_3$ sont tels que définis dans la revendication 1, ou d'un de ses sels, caractérisé en ce que:

a) on prépare un peptide dicyclique bloqué correspondant, fixé à la résine en phase solide, en utilisant, à titre d'un des groupements aminoacides dans la synthèse en phase solide, un dipeptide cyclique ayant la structure IIa ou IIb

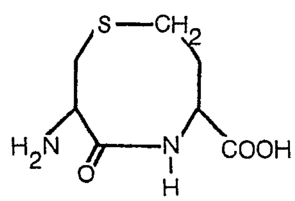

ou

IIa

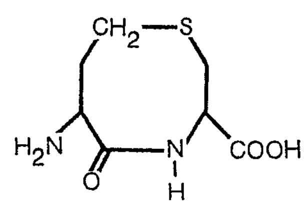

IIb

b) on élimine ensuite le groupe amino N-terminal du peptide monocyclique lié à la résine, puis

c) on sépare de la résine le peptide monocyclique,

d) on traite le peptide monocyclique avec un agent de cyclisation pour former la liaison amide de l'hexapeptide dicyclique recherché, et

e) on élimine les groupes protecteurs de chaînes latérales et on isole le peptide dicyclique de formule Ib, ou un de ses sels.

9. Dipeptide monocyclique répondant aux formules IIa ou IIb

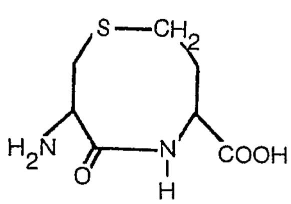

ou

IIa

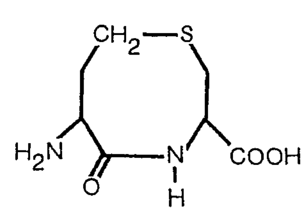

IIb

et dérivés protégés des dipeptides monocycliques de formules IIa et IIb.

10. Composition pharmaceutique, caractérisée en ce qu'elle comprend, comme substance active, un hexapeptide dicyclique de formule I selon la revendication 1 ou un sel non toxique du composé de formule I.